# NEW EUROPEAN PATENT SPECIFICATION

(11) **EP 0 385 476 B2**
(45) Date of publication and mention of the opposition decision: **22.09.1999**
(45) Mention of the grant of the patent: 12.07.1995
(21) Application number: 90104021.2
(22) Date of filing: 01.03.1990
(51) Int. Cl.: C12N 15/16, A61K 38/00, C07K 14/00, C12P 21/02

(54) **Physiologically active polypeptide and DNA**
Physiologisch aktives Polypeptid und DNA
Polypeptide physiologiquement actif et son DNA

(30) Priority: 01.03.1989 JP 4963689; 10.03.1989 JP 5918389
(43) Date of publication of application: 05.09.1990
(73) Proprietor: SHIONOGI & CO., LTD., Osaka 541-0045 (JP); Matsuo, Hisayuki, Kobe-shi, Hyogo (JP)
(72) Inventor: Sudoh, Tetsuji, c/o Daiichi Pure Chemicals Co.,Ltd, Chuo-ku, Tokyo (JP); Maekawa, Keiji, c/o Daiichi Pharm. Res. Institute, Edogawa-ku, Tokyo (JP); Minamino, Naoto, Neyagawa, Osaka 572 (JP); Kangawa, Kenji, Kiyotake-cho, Miyazaki-gun, Miyazaki (JP); Matsuo, Hisayuki, Kobe-shi, Hyogo (JP); Izumi, Atsushi, c/o Daiichi Pure Chem. Co., Ltd., Sumida-ku, Tokyo (JP); Takashima, Mika, c/o Daiichi Pure Chem. Co., Ltd., Sumida-ku, Tokyo (JP)
(74) Representative: Wächtershäuser, Günter, Prof. Dr.

(56) References cited:
- WO-A-89/12069
- BIOCHEMICAL AND BIOPHYSICAL RESEARCH COMMUNICATIONS, vol. 159, no. 3, 31st March 1989, pages 1427-1434, Academic Press, Inc.; T. SUDOH et al.: "Cloning and sequence analysis of cDNA encoding a precursor for human brain natriuretic peptide"
- FEBS LETTERS, vol. 259, no. 2, 1st January 1990, pages 341-375, Federation of European Biochemical Societies, Amsterdam, NL; Y. KAMBAYASHI et al.: "Isolation and sequence determination of human brain natriuretic peptide in human atrium"
- BIOCHEMICAL AND BIOPHYSICAL RESEARCH COMMUNICATIONS, vol. 155, no. 2, 15th September 1988, pages 733-739, Academic Press, Inc.; S. UEDA et al.: "Regional distribution of immunoreactive brain natriuretic peptide in porcine brain and spinal cord"
- BIOCHEMICAL & BIOPHYSICAL RESEARCH COMMUNICATIONS, vol. 157, no. 1, 30th November 1988, pages 402-409, Academic Press, Inc., Duluth, MN, US; N. MINAMINO et al.: "Isolation and identification of a high molecular weight brain natriuretic peptide in porcine cardiac atrium"
- Nature, 1988, Vol. 322, pp.78-81; Sudoh P.
- Biochem. Biophys. Res. Comm., 1988, vol. 157, pp. 402-409; Minamino, N. et al
- Biochem. Biophys. Res. Comm., 1988, vol. 152, pp. 837-842; Kurihara et al
- J. Biol. Chem., 1988, vol. 263, pp. 3850-3857; Newgard et al
- Proc. Natl. Acad. Sci. USA, 1987, vol. 84, pp. 6682-6686; McTiernan et al
- J. Neurochem., 1987, vol. 49, pp. 246-252; Banner et al

## Description

### BACKGROUND OF THE INVENTION

### Field of the Invention:

This invention relates to a physiologically active polypeptide, a DNA encoding the polypeptide, and a pharmaceutical composition for treating and curing circulation diseases comprising the polypeptide as an effective ingredient.

### Description of the Background Art:

Structures of new polypeptides secreted by human or rat artium and having natriuretic activity have successively been determined and reported in the years 1983-1884 *[Biochem. Biophys. Res. Commun. 117,* 859 (1983); *Biochem. Biophys. Res. Commun.* *118*, 131-139 (1984)]. These polypeptides were named artium natriuretic peptides (hereinafter referred to as "ANP"). Since they have strong natriuretic activity as well as relaxing activity of vessel and smooth muscle, they are attracting much attention as a new peptide medicine for circulation disease.

In 1988, a new peptide having diuretic activity was isolated in a purified form from porcine brain. Its structure was determined and the peptide was named "porcine brain natriuretic peptide" (hereinafter referred to as porcine BNP or pBNP) *[Nature, 332,* No. 6159, 78-81 (1988); *Biochem. Biophys. Res. Commun.* *155*, 726-732 (1988)]. Pharmaceutical activities of pBNP resemble those of ANP, and include diuretic activity, natriuretic activity, vasodepressor activity, chicken rectum relaxation activity, and the like. The specific activities of pBNP also resemble those of ANP, except that the rectum relaxation activity of pBNP is 3 to 4 times higher than that of ANP. This is the reason that pBNP is expected to be a new medicine for circulation disease and that studies involving DNA of porcine BNP are being undertaken. Cloning of cDNA possessing a base sequence encoding porcine BNP and its precursor has been reported *[Biochem. Biophys. Res. Commun. 157(1),* 410-416 (1988)].

Development of brain natriuretic peptide derived from human being (hereinafter referred to as human BNP or hBNP) has been desired as a therapeutic agent for human circulation diseases. Such a peptide, however, has not been heretofore found. Nor has its structure been clarified neither on a DNA level nor on a peptide or protein level.

WO 89/12069 corresponding to EP-A-0 418 308 describes the DNA sequences and deduced amino acid sequences of the pre-pro-protein and of the pro-protein of a human natriuretic peptide

In view of this situation, the present inventors have conducted extensive studies to obtain human BNP, and have been successful in cloning cDNA encoding human BNP by screening a cDNA library.

Furthermore, the present inventors have synthesized various human BNPs based on the amino acid sequence deduced from the cDNA base sequence and have studied their pharmacological activities. As a result, the inventors have found that these BNPs had excellent smooth muscle relaxation and natriuretic activities.

### SUMMARY OF THE INVENTION

Accordingly, an object of this invention is to provide a DNA fragment comprising a base sequence encoding a polypeptide derived from human brain and possessing natriuretic activity.

Another object of the present invention is to provide a pharmaceutical composition for curing circulation diseases which comprise as an effective ingredient a physiologically active polypeptide represented by the formula (I), 
X-Cys-Phe-Gly-Arg-Lys-Met-Asp-Arg-Ile-Ser-Ser-Ser-Ser-Gly-
Leu-Gly-Cys-Lys-Val-Leu-Arg-Arg-His-OH (I)
 wherein X is H, H-Gly-Ser-Gly-, or H-Ser-Pro-Lys-Met-Val-Gln-Gly-Ser-Gly-.

Other objects, features and advantages of the invention will hereinafter become more readily apparent from the following description.

### BRIEF DESCRIPTION OF THE DRAWINGS

Figure 1 shows the base sequence determination strategy of cDNA fragment inserted into positive clone hBNP-57 and the restriction endonuclease map of the cDNA.

Figure 2 shows the cDNA base sequence and the amino acid sequence deduced from the base sequence which was determined by the strategy shown in Figure 1.

The Figures 3-A shows the change in relaxation length with time of a chicken rectum specimen to which human BNP-26 of the present invention was administered. Figure 3-B shows the change in relaxation length with time of a chicken rectum specimen to which human BNP-26 of the present invention was administered.

### DETAILED DESCRIPTION OF THE INVENTION AND PREFERRED EMBODIMENTS

In this specification, the peptide of formula (I) having H for X may be referred to as human BNP-23, that having H-Gly-Ser-Gly- for X may be referred to as human BNP-26, and that having H-Ser-Pro-Lys-Met-Val-Gln-Gly-Ser-Gly- for X may be referred to as human BNP-32.

The DNA fragment of the present invention can be prepared, for example, by the following process.

The total RNA is separated from a human tissue which is considered to contain human BNP. mRNA is isolated from the total RNA and a cDNA library is constructed by a conventional method. A DNA fragment encoding human BNP can be isolated by screening the cDNA library by means of hybridization with a probe which has DNA sequence encoding a part of porcine BNP. The process is illustrated in more detail.

### (1) Construction of cDNA library

The mRNA is prepared from tissue such as human brain, human artium, or the like. The RNA can be separated by homogenizing a human artium, for example, by homogenizing the artium together with guanidylthiocyanate, followed by equilibrated density gradient ultracentrifugation using cesium trifluoro acetate. The mRNA is purified according to a conventional manner using oligo (dT) cellulose column chromatography. Synthesis of cDNA from the mRNA is carried out according to a conventional method, e.g. the method using a cDNA synthesis kit (manufactured by Pharmacia Co.), the Okayama-Berg method *(Mol. Cell. Biol. 2*, 161-170, 1982), the method of Gubler, U. and Hoffman, B. J. *(Gene, 25,* 263-269 (1983) or its modification, or a method using other commercially available kit. An endonuclease EcoRI adaptor is applied to cDNA thus obtained, following which the 5'-end is phosphorylated using T-4 polynucleotide kinase or the like. The cDNA library is then constructed by ligation using a vector, e.g. gt10, followed by *in vitro* packaging using, for example, Gigapack Gold (trademark: manufactured by Stratgene Co.).

### (2) Screening of human BNP clone

Screening of human BNP clone is carried out using a labeled porcine cDNA fragment as a probe. This cDNA fragment is, for example, a 120bp fragment which is obtained by the digestion of the complete clone or the incomplete clone obtained in the course of cDNA cloning of the porcine BNP using endonucleases Xhol and Rsal *[Biochem. Biophys. Res. Commun. 157,* 410 (1988)]. The 120bp fragment comprises DNAs encoding the active portion of the porcine BNP (BNP-26) consisting of 26 amino acids and its upstream 30bp. A probe to be used is prepared by labeling this cDNA fragment with ³²P. The probe and the cDNA library prepared in (1) above are hybridized and the positive clone is selected. The DNA fragment encoding human BNP can be prepared by cleaving the selected positive clone with a suitable endonuclease.

To determine the base sequence of the DNA fragment thus obtained the following conventional method is used, for example, by incorporating the DNA fragment into a vector for sequencing, preparing a restriction endonuclease map of the cDNA region, and further incorporating DNA fragments produced by using endonucleases which can cut the DNA into a suitable length into a vector for sequencing to obtain a subclone, and determining the whole base sequence by the method of Sanger et al. *[Proc. Natl, Acad. Sci. USA, 74*, 5463-5469 (1977)].

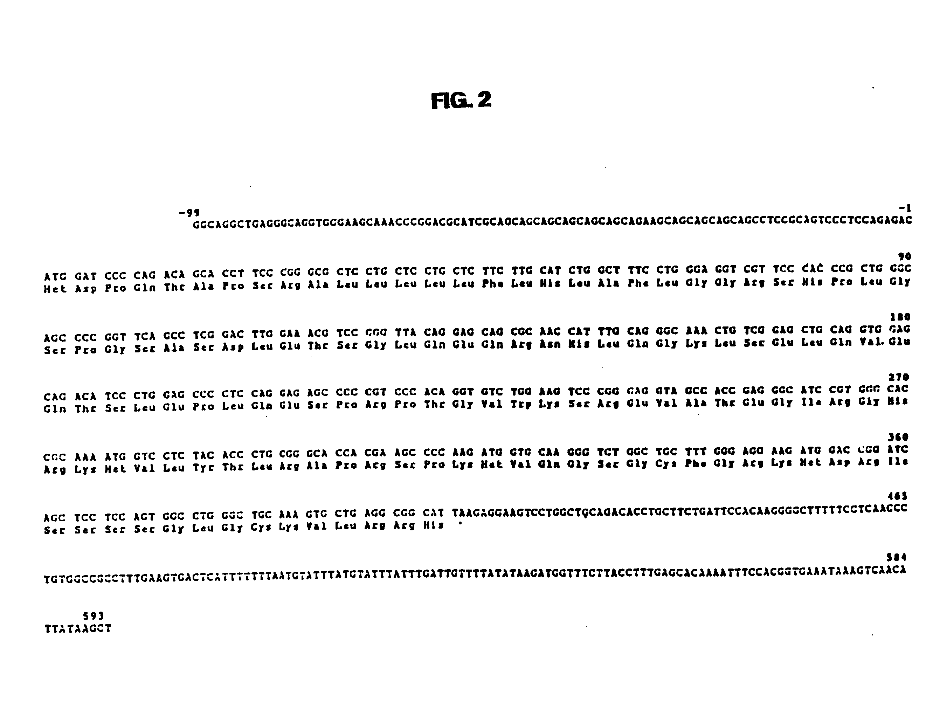

The base sequence of the DNA fragment encoding human BNP thus determined and the amino acid sequence of the human BNP are shown in Figure 2. In the base sequence in Figure 2, the sequence 1-402 is considered to code for pre-pro-human BNP which is comprised of 134 amino acids, and 79-402 of these bases are considered to code for pro-human BNP which is comprised of 108 amino acids. These supposition were based on the fact that the structure of the signal peptide located before the precursor is very similar to that of porcine, and that the porcine BNP also has the amino acid sequence Arg-Ser-His-Pro-Leu-Gly corresponding to the base numbers 73-90 and the Ser-His bond of this sequence is cut to form the porcine BNP precursor *[Biochem. Biophys. Res. Commun. 157,* 410 (1988)]. Among these, sequence 307-402 is considered to encode the human BNP-32 which is comprised of 32 amino acids. The precursor is considered to form the human BNP-32 as a result of the processing, although this is not decisive because the human BNP has never been successfully isolated as a peptide. Of porcine DNAs, porcine BNP-26 *[Nature, 332,* 78-81 (1988)] and porcine BNP-32 consisting of 32 amino acids *[Biochem. Biophys. Res. Commun. 155*, 726-732 (1988)] have been isolated. As for ANP, human aANP consisting of 28 amino acids from human tissue *[Biochem. Biophys. Res. Commun. 118,* 131-139 (1984)] and rat aANP consisting of 28 amino acids from rat tissue *[Biochem. Biophys. Res. Commun. 117,* 859-865 (1983)] have been isolated. All of them are produced by the processing which took place downstream of Arg and Pro-Arg existing in the precursor. In the case of human BNP, since an Arg preceding the peptide having 23 amino acids from Cys (112) to His (134) which is considered to exhibit activity is the 102 Arg and this Arg has a Pro-Arg structure, the human BNP-32 consisting of 32 amino acids is presumed to be produced as a result of the processing after the Pro-Arg structure.

Based on the fact that the ANP precursor had been found to have a physiological activity, the human BNP precursor is also considered to have some physiological activity. Thus, both the human BNP-32 and the precursor are useful as a medicine.

The DNA corresponding to the base number 1-402 and the amino acid sequence deduced from the base sequence are as shown in Figure 2.

The peptide of the present invention which is represented by formula (I) can also be prepared by the solid phase method or the liquid phase method which are conventionally used in the art [e.g. N. Izumiya, et al. "Peptide Synthesis", Maruzen Publishing Co., Ltd. (1984); "Lecture of Biochemistry Experiment (I), Protein Chemistry" edited by Chemical Society of Japan, vol. 1, 208-495 (1977), published by Tokyo Kagaku Dojin].

When the peptide (I) is prepared by the solid phase method, the following protective groups of amino acid can preferably be used; i.e., 9-fluorenylmethyloxycarbonyl (Fmoc) group for the α-amino group, tert-butyl (tBu) group for the β-carboxyl group of aspartic acid, 4-methoxy-2,3,6-trimethylbenzenesulfonyl (Mtr) group for the guanidino group of arginine, tert-butyl (tBu) group for the hydroxyl group of serine, acetamidomethyl (Acm) group for the thiol group of cysyeine, trityl group (Trt) for the imidazole group of histidine, and tert-butyloxycarbonyl (Boc) group for the ε-amino group of lysine. *p*-Alkoxybenzyl alcohol resin (Wang Resin) is a preferable insoluble resin for use. Preferable methods used for the condensation of protected amino acids are the dicyclohexylcarbodiimide (DCC) method, the active ester method using 1,3-diisopropylcarbodiimide (DIC), the acid anhydride method using DCC, the diphenylphosphoryl azide (DPPA) method, and the like. The protective groups are not limited to those given above. α-amino group of amino acids, for example, may be protected by tert-butyloxycarbonyl (Boc) group.

Production of the polypeptide of the present invention by the solid phase method can be carried out, for example, by the following manner. The protected derivative Fmoc-His(Trt)-OH in which His is the C-terminal amino acid of the polypeptide is first introduced into *p*-alkoxybenzyl alcohol resin. The corresponding protected amino acids are successively combined in this way to synthesize a protected peptide resin. Subsequently, removal of peptide from the resin and elimination of protective groups other than Acm are concurrently performed by the treatment with piperidine and trifluoroacetic acid (TFA), the treatment with piperidine and trimethylsilyl bromide (TMSBr) *[Chem. Pharm. Bull., 35*, (9), 3880 (1987)], and the like to obtain a peptide having an Acm group for thiol of cystein, such a peptide being referred to as Cys(Acm)-peptide. Then, the Cys(Acm)-peptide is oxidized with iodine to remove the thiol protective group and, at the same time, to form a disulfide bond between two thiol groups of cystein in the peptide molecule, thus producing crude polypeptide of the present invention.

The crude polypeptide is purified by a conventional manner such as, for example, gel filtration, ion exchange chromatography, reversed phase HPLC, or the like.

The peptide of formula (I) of the present invention can be converted into an acid addition salt according to a conventional manner using an inorganic acid such as hydrochloric acid, sulfuric acid, phosphoric acid, or the like; or an organic acid such as formic acid, acetic acid, citric acid, tartaric acid, fumaric acid, maleic acid, or the like.

The peptide of formula (I) of the present invention thus produced possesses smooth muscle relaxation and other activities.

### <Smooth muscle relaxation activity>

### (1) Test method

Rectum of a chicken, age 4-7 days, was enucleated and muscle specimens, 1.5 cm long, were prepared. The specimens were immersed into 2.5 ml of Krebs-Henseleit solution, containing 2 x 10⁸ M carbachol, which was aerated with a 95% O₂-5% CO₂ mixed gas and maintained at 32°C in a 3 ml organ bath. The specimens were allowed to equilibrate for about 30 minutes. When the self-acting of the muscle became constant, 100 ng of human BNP-26 was added and relaxation of the muscle was measured for 6-8 minutes after the addition. After the measurement, the specimens were rinsed two or three times, and, after 20-30 minutes, the above procedure was repeated using 200 ng of human BNP-32. The human BNPs were used dissolved in a prescribed amount of physiological saline. Isotonic relaxations of the muscles under a tension of 0.5 g were registered on a kymograph (KN-259: trademark, product of Natsumeseisakusyo Co.)

### (2) Results

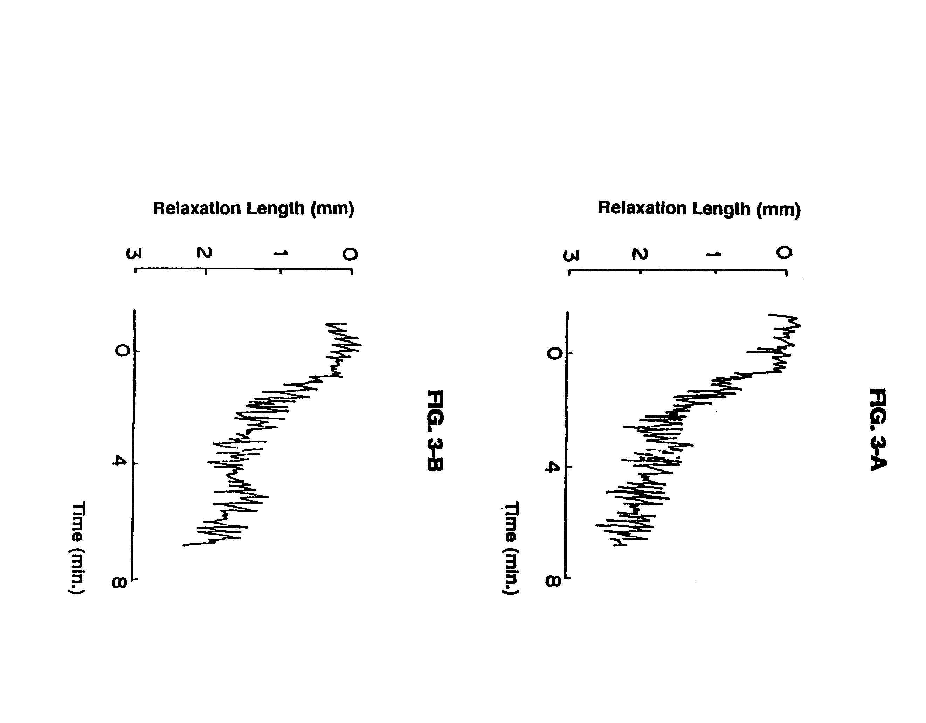

The results are shown in Figures 3-A and 3-B. From the results, the polypeptide of the present invention was found to exhibit strong smooth muscle relaxation activity at a dose of 100-200 ng.

As mentioned above, the human BNP produced by the present invention possesses excellent smooth muscle relaxation activity, diuretic or natriuretic activity, and vasodepressor activity. The BNP is safe as a medicine for humans because it is derived from human, thus it can be used as a medicine for curing such diseases as cardiac edema, nephric edema, hepatic edema, pulmonary edema, hypertension, congestive heat failure, acute and chronic renal failure, and the like.

Any methods conventionally used for the administration of peptide medicines, e.g. intravenous injection, intramuscular injection, subcutaneous injection, sublingual administration, intracutaneous administration, rectum administration, or the like, can be employed for the administration of the peptide of the present invention.

A preferable dose is 0.5 µg/kg to 100 mg/kg, with the especially preferable range being 0.5 µg/kg to 1 mg/kg.

Other features of the invention will become apparent in the course of the following description of the exemplary embodiments which are given for illustration of the invention and are not intended to be limiting thereof.

### EXAMPLES

### Example 1

### (1) Construction of cDNA library

Human artium (3 g) was pulverized by a treatment with with liquid nitrogen. To this was added, according to the method of Chirgwin et al. [Chirgwin, J. W. et al. *Biochemistry, 18,* 5294-5299 (1979)], a guanidiumthiocyanate aqueous solution, and the mixture was homogenized. The whole RNA was separated by equilibrium density gradient ultracentrifugation using cesium trifluoro acetate, following which the RNA was purified, according to a conventional method, by oligo (dT) cellulose column chromatography to isolate 37 ug of poly(A)⁺RNA(mRNA).

cDNA was synthesized from 3 µg of mRNA by using a cDNA synthesis kit (product of Pharmacia Co.). After the addition of an EcoRI adaptor, the 5'-end was phosphorylated with T4 polynucleotide kinase and ligation was carried out using Xgtl0 as a vector. λgt10 arms which was digested by EcoRI and dephosphorylated was employed as a vector. For 2 µg of the λgt10, the amount of cDNA used for the ligation was 0.1 µg converted to the amount of RNA used for the cDNA synthesis. After the ligation, the product was packed using a packaging kit (Gigapack Gold, product of Stratgene Co.), and cDNA library was obtained.

A small amount of cDNA was inoculated into *E. coli* c600 and c600hfl in order to investigate the completeness of the cDNA library. As a result, it was found that among the plaque produced by *E. coli* c600, 90% was transparent and 10% was turbid, evidencing that the share of the recombinant phage in the library was 90%. Furthermore, the number of plaques in the whole cDNA library was found to be 7 x 10⁷.

### (2) Screening of human BNP clone

Screening was carried out on 5 x 10⁵ plaques expressed by inoculating a portion of cDNA library into *E. coli* c600hfl. The cDNA fragment encoding porcine BNP, which was labeled with ³²P, was used as a probe for the screening. The cDNA fragment was prepared from plasmid BNP-82 (320 bp) which is an incomplete clone obtained in the course of cDNA cloning of the porcine BNP *[Biochem. Biophys. Res. Commun. 157*, 410 (1988)], and plasmid BNP-82 was digested by restriction endonucleases Xhol and Rsal to obtain a 120 bp fragment which consists of a DNA fragment encoding porcine BNP-26 and its upstream 30 bp. The 120 bp fragment was then purified by acrylamide gel electrophoresis.

Plaques were first transferred to a nylon filter, and neutralized after alkali treatment, following which DNA was fixed by UV irradiation. The filter was immersed into a 5 x Denhardts solution and a 4 x SSC solution of 0.6 M NaCl and 0.06 M sodium citrate containing 100 µg/ml of denatured salmon sperm DNA and 0.1% SDS at 60°C for 3 hours, thus effecting hybridization. A probe labeled with ³²P by a Random primed DNA labeling kit (Product of Belinger Manheim Co.) was added to the hybridization solution having the same composition as the prehybridization solution to a concentration of 2 x 10⁶ cpm/ml, wherein the filter was incubated overnight at 60°C.

The filter was then washed with a 2 x SCC solution containing 0.1% SDS, dried in the air, and submitted to autoradiography.

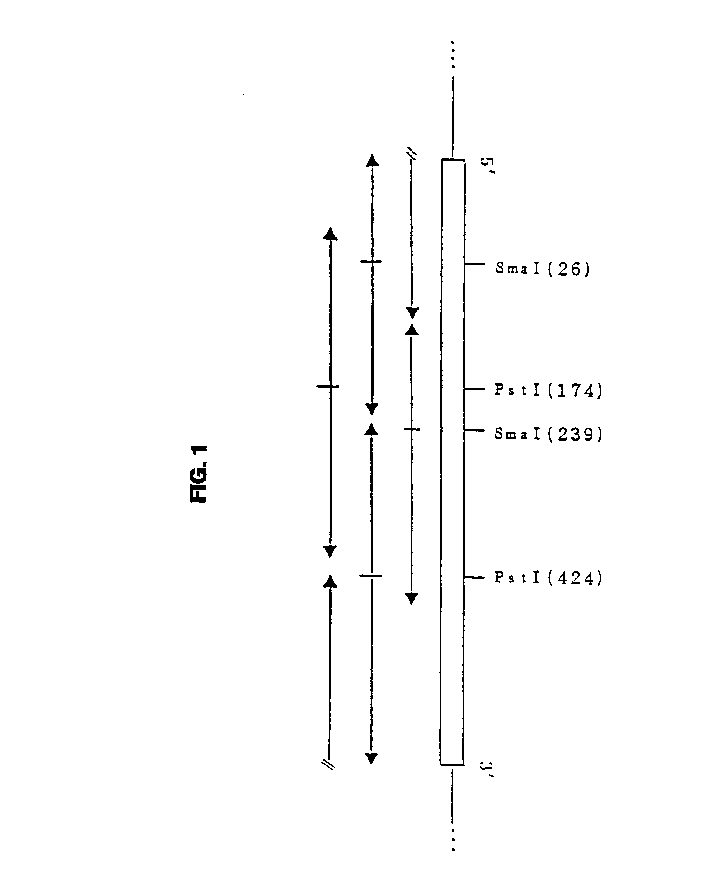

Fifty five (55) hybridization positive plaques were thus obtained. The 55 positive plaques were submitted to a test to detect whether they could hybridize using the DNA (680 bp) encoding human ANP as a probe, and were found that all were negative. This is an evidence that this cDNA is different from the known cDNA encoding human ANP. The above 55 positive plaques were monocloned and λ-phage DNA was prepared according to a conventional method. A DNA fragment obtained by cleaving the λ-phage DNA with the restriction endonulease EcoRI was investigated and was found that a cDNA having a muximum length of about 700 bp was inseted into a clone which was named λhBNP-57. This insert cDNA was incorporated into Blue Script (KS (+)) (product of Stratgene Corp.) which is a sequencing vector, thus producing phBNP-57. The *E. coli* containing this plasmid was named *E. coli* HB101/phBNP-57 and deposited with Fermentation Research Institute, Agency of Industrial Science and Technology (Deposition No. 2299, FERM BP-2299). A restriction endonulease map of the cDNA region was prepared. cDNA fragments were prepared using suitable restriction endonuleases which could cut the cDNA into a suitable length and these fragments were again incorporated into the blue script and subcloned. Figure 1 shows the restriction endonulease cleaving sites of the cDNA region and the base sequence determination strategy. The base sequence was determined by the method of Sanger et al. *[Proc. Natl. Acad. Sci.,* USA, 74, 5463-5467 (1977)],

Figure 2 shows the cDNA base sequence and the amino acid sequence corresponding to the base sequence.

The inserted DNA sequence has a long translational region starting from a translation initiation codon, ATG, and ending a translation termination codon, TAA. The cDNA having the whole length of 692 bp is considered to have a 5'-side non-translational region of the base pair number -99 to -1, 1-78 codes for a signal peptide, and 79-402 codes for the human BNP precursor. Among these, 307-402 are considered to codes for the human BNP-32 consisting of 32 amino acids 403-593 are a non-translational region.

The amino acid sequence corresponding to the base sequence of 307-402 encoding the human BNP-32 has a cyclic structure which is formed with the cysteine disulfide bond of 17 amino acids and is very similar to porcine BNP.

### Example 2

A pro-human-BNP-producing vector can be obtained by using phBNP-57 clone, a recombinant plasmid which is constructed by insertion of a cDNA obtained by digestion of λphBNP-57 with the restriction endonuclease EcoRI into a plasmid blue script. More specifically, a new restriction endonuclease recognition site and a translation initiation codon (ATG) can be introduced at the site immediately preceding the pro-human-BNP-code region of phBNP-57 by site-directional mutation. A fragment is isolated by the utilization of this new recognition site. The above fragment is then inserted into the expression vector at immediately downstream of the plasmid promoter, and the plasmid is inserted into *E. coli.* The *E. coli* is cultured with nutrients sufficient to synthesize polypeptide followed by which pro-human BNP is collected. Another method of obtaining the human BNP-32 or the fragment to be cultured by *E. coli* is changing the site of site-directional mutation and the base sequence on the inserted cDNA of phBNP-57. The human BNP-32 or the fragment is then inserted into the expression vector and the vector is introduced into *E. coli* The human BNP-32 or the human BNP is obtained from the cultured *E. coli.*

### Example 3

### (1) Synthesis of peptide human BNP-26 and human BNP-32

### (a) Synthesis of a protected peptide resin

For the synthesis of the protected peptide resin all α-amino groups of amino acids were protected by 9-fluorenylmethyloxycarbonyl (Fmoc) group, and among active side chains, the β-carboxyl group of aspartic acid was protected by tert-butyl (tBu) group, the guanidino group of arginine was protected by 4-methoxy-2,3,6-trimethylbenzenesulfonyl (Mtr) group, the hydroxyl group of serine was protected by tert-butyl (tBu) group, the thiol group of cysteine was protected by acetamidomethyl (Acm) group, the imidazole group of histidine was by trityl group (Trt), and the ε-amino group of lysine was by tert-butyloxycarbonyl (Boc) group. 1.0 g of *p*-alkoxybenzyl alcohol resin into which Fmoc-His(Trt) group was introduced was used as the resin.

In the condensation of the protected amino acid, the Fmoc group which is the protected group for the N-terminal amino acid of the protected peptide bonding to the resin was almost completely removed by the treatment with piperidine, repeated twice, at room temperature for 6 minutes. The free amino group from which the Fmoc group was eliminated was condensed with the carboxyl group of the Fmoc protected amino acid located next in the sequence of the target peptide. The condensation of the protected amino acid was carried out by treating 1 mmol of Fmoc-protected amino acid with 1,3-diisopropylcarbodiimide (DIC) in the presence of 1-hydroxybenztriazole. The same procedure was repeated when the reaction was not completed by this treatment. The progress and completion of the reaction were monitored by the Keizer test using ninhydrin. 
Fmoc-Gly-Ser(tBu)-Gly-Cys(Acm)-Phe-Gly-Arg(Mtr)-
Lys(Boc)-Met-Asp(tBu)-Arg(Mtr)-Ile-Ser(tBu)-Ser(tBu)-
Ser(tBu)-Ser(tBu)-Gly-Leu-Gly-Cys(Acm)-Lys(Boc)-Val-Leu-
Arg(Mtr)-Arg(Mtr)-His(Trt)- resin
 was thus synthesized. At this stage, a portion of the product was taken out and the Fmoc group was removed in the same manner as described above, thus obtaining 670 mg of 
H-Gly-Ser(tBu)-Gly-Cys(Acm)-
Phe-Gly-Arg(Mtr)-Lys(Boc)-Met-Asp(tBu)-Arg(Mtr)-Ile-
Ser(tBu)-Ser(tBu)-Ser(tBu)-Ser(tBu)-Gly-Leu-Gly-Cys(Acm)-
Lys(Boc)-Val-Leu-Arg(Mtr)-Arg(Mtr)-His(Trt)- resin
 (hereinafter referred to as "protected human BNP-26 resin").

The remaining resin was further subjected to N-terminal extension reaction to obtain

Fmoc-Ser(tBu)-Pro-Lys(Boc)-
Met-Val-Gln-Gly-Ser(tBu)-Gly-Cys(Acm)-Phe-Gly-Arg(Mtr)-
Lys(Boc)-Met-Asp(tBu)-Arg(Mtr)-Ile-Ser(tBu)-Ser(tBu)-
Ser(tBu)-Ser(tBu)-Gly-Leu-Gly-Cys(Acm)-Lys(Boc)-Val-Leu-
Arg(Mtr)-Arg(Mtr)-His(Trt)- resin.

The Fmoc group was removed in the same manner as described above, thus obtaining 1.5 g of 
H-Ser(tBu)-Pro-Lys(Boc)-Met-Val-
Gln-Gly-Ser(tBu)-Gly-Cys(Acm)-Phe-Gly-Arg(Mtr)-Lys(Boc)-
Met-Asp(tBu)-Arg(Mtr)-Ile-Ser(tBu)-Ser(tBu)-Ser(tBu)-
Ser(tBu)-Gly-Leu-Gly-Cys(Acm)-Lys(Boc)-Val-Leu-Arg(Mtr)-
Arg(Mtr)-His(Trt)- resin
 (hereinafter referred to as "protected human BNP-32 resin").

### (b) Synthesis of Cys(Acm)-human BNP-26

The protected human BNP-26 resin (600 mg) was deprotected with 2.4 ml of thioanisole, 20 ml of trifloroacetic acid (TFA), 2.6 ml trimethylsilyl bromide (TMSBr), and 340 µl of ethanedithiol at 0°C for 3 hours. After the reaction, the resultant reaction mixture was washed with 200 ml of ether to remove anisole, and the product was extracted with 20 ml of 1 N acetic acid. The resin and the insoluble substance was removed by centrifugation. To the residue was added 1 ml of 1 M sodium fluoride (NaF) with cooling. The mixture was adjusted to pH 8 with 5% aqueous ammonia using a Universal test paper and left for 30 minutes. After adjusting to pH 5 with 1 N acetic acid, the mixture was diluted with water to a volume of 10-fold, absorbed to a column (φ 3 cm x 8.5 cm) packed with 60 ml of ODS resin (LC-Sorb: trademark, product of Chemco Co.), washed thoroughly with 0.1 N acetic acid, and eluted with 200 ml of 60% acetonitrile containing 0.1% TFA. The acetonitrile was evaporated under reduced pressure and the residue was freeze-dried to obtain 300 mg of crude Cys(Acm)-human BNP-26.

The crude product was dissolved into 9 ml of 1 N acetic acid and the solution was subjected to reversed phase HPLC over a nucleosil 120-5C18 column (20 x 250 mm) in 9 portions at a flow rate of 5 ml/min. Solvent (A), a mixture of water:acetonitrile:10% TFA = 90:10:1, and Solvent (B) a mixture of water:acetonitrile:10% TFA = 40:60:1, were used at a linear gradient from (A):(B)=90:10 to (A):(B)=55:45 for 120 minutes. This procedure was repeated 9 times and the main peak eluted at 57-61 minute was collected. Acetonitrile was evaporated from the collected fraction and the residue was freeze-dried to obtain 96.0 mg of Cys(Acm)-human BNP-26.

### (c) Synthesis of human BNP-26

Solution A was prepared by dissolving 227 mg of iodine into 50 ml of 95% acetic acid and by adding 80 µl of 1 N hydrochloric acid.

Solution B was prepared by dissolving 2.1 g of citric acid and 575 mg of L-ascorbic acid into 10 ml of 2 N sodium hydroxide, and made up to the final volume of 50 ml by an addition of water.

A solution 89.0 mg of Cys(Acm)-human BNP-26 in 5 ml of 90% acetic acid was added dropwise into 30 ml of Solution A at room temperature while stirring. After the addition, the mixture was stirred for a further 20 minutes. To this mixture Solution B was added dropwise until the brown color of iodine disappeared. The resulting solution was diluted with 500 ml of water and applied to a column (φ 2 cm x 9.5 cm) packed with 30 ml of ODS resin (LC-Sorb: trademark, product of Chemco Co.). The column was washed thoroughly with 0.1 N acetic acid, and eluted with 60 ml of 60% acetonitrile containing 0.1% TFA. The acetonitrile was evaporated under reduced pressure and the residue was freeze-dried to obtain 60.0 mg of crude human BNP-26.

The crude product was dissolved into 4 ml of 1 N acetic acid and the solution was applied to reversed phase HPLC over a nucleosil 120-5C18 column (20 x 250 mm) in 4 divided portions at a flow rate of 5 ml/min. Linear gradient elution was carried out using Solvent (A), a mixture of water:acetonitrile:10% TFA = 90:10:1, and Solvent (B), a mixture of water:acetonitrile:10% TFA = 40:60:1, from (A):(B)=90:10 to (A):(B)-= 55:45 for 120 minutes. This procedure was repeated 4 times and the main peak eluted at 62-66 minute was collected. Acetonitrile was evaporated from the collected fraction and the residue was freeze-dried to obtain 25 mg of human BNP-26.

### (d) Synthesis of Cys(Acm) human BNP-32

Release from the resin and removal of the protected group was performed in the same manner as described in (b) on 700 mg protected human BNP-32 using thioanisole, TFA, TMSBr, and ethanedithiol. The product was purified over reversed phase HPLC to obtain 60.0 mg of Cys(Acm)-human BNP-32.

### (e) Synthesis of human BNP-32

60.0 mg of Cys(Acm)-human BNP-32 was subjected to the Acm removal and cyclization in the same manner as (c) using iodine to obtain 20.0 mg of crude human BNP-32. The crude product was dissolved into 4 ml of 1 N acetic acid and the solution was subjected to reversed phase HPLC over a nucleosil 120-5C18 column (20 x 250 mm) in 4 portions at a flow rate of 5 ml/min. Elution was carried out linear gradiently using Solvent (A), a mixture of water:acetonitrile:10% TFA = 90:10:1, and Solvent (B) a mixture of water:acetonitrile:10% TFA = 40:60:1, from (A):(B)=90:10 to (A):(B)=55:45 for 120 minutes. This procedure was repeated 4 times and the main peak eluted at 61-64 minute was collected. Acetonitrile was evaporated from the collected fraction and the residue was freeze-dried to obtain 5 mg of human BNP-32.

### (2) Physicochemical Characteristics

Physicochemical characteristics of human BNP-26 and human BNP-32 prepared in (1) above were as follows.
(a) Form
White powder
(b) Solubility in solvents
Soluble in water, acidic aqueous solutions, and acetic acid. Insoluble in chloroform, benzene, ethyl ether, and hexane.
(c) Property
basic
(d) Amino acid composition
Given in Table 1.

**TABLE 1**

| Peptide | Human BNP-26 | Human BNP-32 |
|---|---|---|
| Molecular Weight | 2793.28 | 3464.12 |
| Amino Acid Composition * | Measured (Calculated) | Measured (Calculated) |
| Asp + Asn | 1.06 (1) | 0.94 (1) |
| Ser | 4.61 (5) | 4.55 (6) |
| Glu + Gln | - | 0.94 (1) |
| Gly | 5.33 (5) | 4.56 (5) |
| Cys ** | 1.62 (2) | 1.61 (2) |
| Val | 0.90 (1) | 1.81 (2) |
| Met | 1.02 (1) | 1.81 (2) |
| lle | 0.94 (1) | 0.93 (1) |
| Leu | 1.98 (2) | 1.87 (2) |
| Phe | 1.00 (1) | 1.00 (1) |
| Lys | 1.97 (2) | 2.71 (3) |
| His | 1.00 (1) | 0.95 (1) |
| Arg | 3.99 (4) | 3.78 (4) |
| Pro *** | - | 1.08 (1) |

| | | |
|---|---|---|
| * The measured value (mols) in the table is one example of amino acid analysis. | | |
| ** Measured as Cys-SO₃ H after the oxidation with performic acid, followed by hydrolysis. Other amino acids were hydrolyzed with 6 N HCI. | | |
| *** Measured at 440 nm. | | |

## Claims (Claims for the following Contracting State(s): AT, BE, CH, LI, DE, DK, FR, GB, GR, IT, LU, NL, SE)

1. A DNA fragment coding for a polypeptide derived from human brain and possessing natriuretic activity and consisting of the following amino acid sequence:

Ser Pro Lys Met Val Gln Gly Ser Gly Cys Phe Gly Arg
Lys Met Asp Arg Ile Ser Ser Ser Ser Gly Leu Gly Cys
Lys Val  Leu Arg Arg His.

2. The DNA fragment according to Claim 1 having the following base sequence:

AGC CCC AAG ATG GTG CAA GGG TCT GGC TGC TTT GGG AGG AAG
ATG GAC CGG ATC AGC TCC TCC AGT GGC CTG GGC TGC AAA GTG
CTG AGG CGG CAT .

3. A physiologically active polypeptide represented by formula (I) 
X-Cys-Phe-Gly-Arg-Lys-Met-Asp-Arg-Ile-Ser-Ser-Ser-Ser-Gly-
Leu-Gly-Cys-Lys-Val-Leu-Arg-Arg-His-OH (I)
 wherein X is H, H-Gly-Ser-Gly-, or H-Ser-Pro-Lys-Met-Val-Gln-Gly-Ser-Gly-.

4. A pharmaceutical composition for curing circulation diseases which comprises as an effective ingredient the physiologically active polypeptide of Claim 3.

## Claims (Claims for the following Contracting State(s): ES)

1. A process for the preparation of a DNA fragment coding for a polypeptide derived from human brain and possessing natriuretic activity and consisting of the following amino acid sequence: 
Ser Pro Lys Met Val Gln Gly Ser Gly Cys Phe Gly Arg
Lys Met Asp Arg Ile Ser Ser Ser Ser Gly Leu Gly Cys
Lys Val  Leu Arg Arg His,
 which process is characterized in that it comprises the following steps:
a) construction of a cDNA library by means of the following operations: (1) isolating mRNA from human brain or human artium by means of homogenization thereof; isolation by ultra-centrifuging and purification; (2) synthesis of the cDNA from the thus obtained mRNA; (3) applying an EcoRI adaptor endonuclease to the cDNA thus obtained and then phosphorylyzing the 5'-end; (4) ligation using a vector whereby the library remains constructed;
b) screening of human BNP clone using a labeled porcine cDNA fragment as a probe or hybridizing both to select the positive clone;
c) cleaving the selected positive clone with a suitable endonuclease to obtain the desired DNA fragment.

2. A process according to claim 1 characterized in that the DNA fragment has the following base sequence:

AGC CCC AAG ATG GTG CAA GGG TCT GGC TGC TTT GGG AGG AAG
ATG GAC CGG ATC AGC TCC TCC AGT GGC CTG GGC TGC AAA GTG
CTG AGG CGG CAT.

3. A process for preparing a physiologically active polypeptide, represented by formula (I): 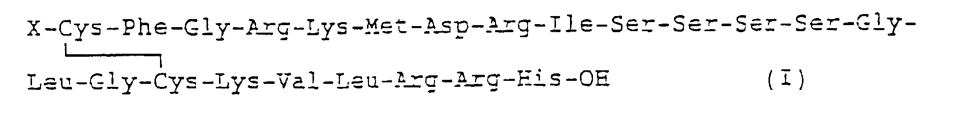
X-Cys-Phe-Gly-Arg-Lys-Met-Asp-Arg-Ile-Ser-Ser-Ser-Ser-Gly-
Leu-Gly-Cys-Lys-Val-Leu-Arg-Arg-His-OH (I)
 wherein X is H, H-Gly-Ser-Gly-, or H-Ser-Pro-Lys-Met-Val-Gln-Gly-Ser-Gly-,
which process is characterized in that it comprises:
a) preparing the polypeptide by means of solid phase techniques by the following manner: (i) the protected derivative Fmoc-His(Trt)-OH in which His is the C-terminal amino acid of the polypeptide is first introduced in p-alkoybenzyl alcohol resin; (ii) the corresponding protected amino acids are successively combined in this way to synthesize a protected peptide resin; (iii) subsequently, removing of polypeptide from the resin and elimination of protective groups other than Acm to obtain a peptide having an Acm group of thiol of cystein, such a peptide being referred to as Cys(Acm)- peptide; (iv) then, the Cys(Acm)-peptide is oxidized with iodine to remove the thiol protective group and, at the same time, to form a disulfide bond between two thiol groups of cystein in the peptide molecule, thus producing crude polypeptide of formula (i) indicated above, which is subsequently purified.

## Patentansprüche (Patentansprüche für folgende(n) Vertragsstaat(en): AT, BE, CH, LI, DE, DK, FR, GB, GR, IT, LU, NL, SE)

1. DNA-Fragment, kodierend für ein Polypeptid, das von menschlichem Hirn abgeleitet ist und natriuretische Wirksamkeit aufweist sowie aus der nachstehenden Aminosäuresequenz besteht:

Ser Pro Lys Met Val Gln Gly Ser Gly Cys Phe Gly Arg
Lys Met Asp Arg Ile Ser Ser Ser Ser Gly Leu Gly Cys Lys Val
Leu Arg Arg His.

2. DNA-Fragment nach Anspruch 1 mit nachstehender Basensequenz:

AGC CCC AAG ATG GTG CAA GGG TCT GGC TGC TTT GGG AGG
AAG ATG GAC CGG ATC AGC TCC TCC AGT GGC CTG GGC TGC AAA GTG
CTG AGG CGG CAT.

3. Physiologisch wirksames Polypeptid, wiedergegeben durch die Formel (I) 
X-Cys-Phe-Gly-Arg-Lys-Met-Asp-Arg-Ile-Ser-Ser-Ser-
Ser-Gly-Leu-Gly-Cys-Lys-Val-Leu-Arg-Arg-His-OH (I),
 wobei X H, H-Gly-Ser-Gly- oder H-Ser-Pro-Lys-Met-Val-Gln-Gly-Ser-Gly- bedeutet.

4. Arzneimittel zur Behandlung von Kreislauferkrankungen, umfassend als einen Wirkstoff, das physiologisch wirksame Polypeptid von Anspruch 3.

## Patentansprüche (Patentansprüche für folgende(n) Vertragsstaat(en): ES)

1. Verfahren zur Herstellung eines DNA-Fragments, das für ein Polypeptid kodiert, das von menschlichem Hirn abgeleitet ist und natriuretische Wirksamkeit aufweist sowie aus der nachstehenden Aminosäuresequenz besteht: 
Ser Pro Lys Met Val Gln Gly Ser Gly Cys Phe Gly Arg
Lys Met Asp Arg Ile Ser Ser Ser Ser Gly Leu Gly Cys Lys Val
Leu Arg Arg His,
 wobei das Verfahren dadurch gekennzeichnet ist, daß es die nachstehenden Schritte umfaßt:
a) Konstruktion einer cDNA-Genbank durch nachstehende Arbeitsgänge: (1) Isolieren von mRNA aus menschlichem Hirn oder menschlichem Artium durch Homogenisieren davon, Isolieren durch Ultrazentrifugieren und Reinigung, (2) Synthese der cDNA aus der so erhaltenen mRNA, (3) Anwenden einer EcoRI-Adaptorendonuclease auf die so erhaltene cDNA und anschließend Phosphorylieren des 5'-Endes, (4) Ligierung unter Verwendung eines Vektors, wodurch die Genbank aufgebaut bleibt,
b) Absuchen nach Human-BNP-Klon unter Verwendung eines markierten Schweine-cDNA-Fragments als Sonde oder Hybridisieren beider zum Auswählen des positiven Klons;
c) Schneiden des ausgewählten positiven Klons mit einer geeigneten Endonuclease zu dem gewünschten DNA-Fragment.

2. Verfahren nach Anspruch 1, dadurch gekennzeichnet, daß das DNA-Fragment nachstehende Basensequenz aufweist:

AGC CCC AAG ATG GTG CAA GGG TCT GGC TGC TTT GGG AGG
AAG ATG GAC CGG ATC AGC TCC TCC AGT GGC CTG GGC TGC AAA GTG
CTG AGG CGG CAT.

3. Verfahren zur Herstellung eines physiologisch wirksamen Polypeptids, wiedergegeben durch die Formel (I) 
X-Cys-Phe-Gly-Arg-Lys-Met-Asp-Arg-Ile-Ser-Ser-Ser-
Ser-Gly-Leu-Gly-Cys-Lys-Val-Leu-Arg-Arg-His-OH (I),
 wobei X H, H-Gly-Ser-Gly- oder H-Ser-Pro-Lys-Met-Val-Gln-Gly-Ser-Gly- bedeutet, wobei das Verfahren dadurch gekennzeichnet ist, daß es umfaßt:
a) Herstellen des Polypeptids mit Hilfe von Festphasentechniken in nachstehender Weise: (i) das geschützte Derivat Fmoc-His(Trt)-OH, worin His die C-terminale Aminosäure des Polypeptids bedeutet, wird zunächst in p-Alkoxybenzylalkoholharz eingeführt; (ii) die entsprechenden geschützten Aminosäuren werden nacheinander in dieser Weise kombiniert zur Synthese eines geschützten Peptidharzes; (iii) anschließend erfolgt Entfernen des Polypeptids vom Harz und Eliminieren der Schutzgruppen außer Acm zu einem Peptid mit einer Acm-Gruppe am Thiol von Cystein, wobei ein derartiges Peptid als Cys(Acm)-Peptid bezeichnet wird; (iv) anschließend wird das Cys(Acm)-Peptid mit Jod unter Entfernung der Thiolschutzgruppe oxidiert und gleichzeitig unter Bildung einer Disulfidbindung zwischen zwei Thiolgruppen von Cystein in dem Peptidmolekül und somit wird rohes Polypeptid der Formel (i), wie vorstehend ausgewiesen, hergestellt, das anschließend gereinigt wird.

## Revendications (Revendications pour l'(les) Etat(s) contractant(s) suivant(s): AT, BE, CH, LI, DE, DK, FR, GB, GR, IT, LU, NL, SE)

1. Fragment d'ADN codant pour un polypeptide issu du cerveau humain, possédant une activité natriurétique et constitué par la séquence d'acides aminés suivante:

Ser Pro Lys Met Val Gln Gly Ser Gly Cys Phe Gly
Arg Lys Met Asp Arg Ile Ser Ser Ser Ser Gly Leu Gly
Cys Lys Val Leu Arg Arg His.

2. Fragment d'ADN selon la revendication 1 ayant la séquence de bases suivante :

AGC CCC AAG ATG GTG CAA GGG TCT GGC TGC TTT GGG AGG
AAG ATG GAC CGG ATC AGC TCC TCC AGT GGC CTG GGC TGC
AAA GTG CTG AGG CGG CAT.

3. Polypeptide physiologiquement actif représenté par la formule (I) 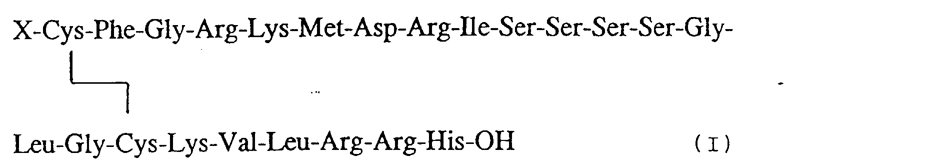
X-Cys-Phe-Gly-Arg-Lys-Met-Asp-Arg-Ile-Ser-Ser-Ser-Ser-Gly-
Leu-Gly-Cys-Lys-Val-Leu-Arg-Arg-His-OH (I)
 dans laquelle X est H, H-Gly-Ser-Gly-, ou H-Ser-Pro-Lys-Met-Val-Gln-Gly-Ser-Gly-.

4. Composition pharmaceutique pour corriger les troubles de la circulation qui comprend à titre de principe actif le polypeptide physiologiquement actif selon la revendication 3.

## Revendications (Revendications pour l'(les) Etat(s) contractant(s) suivant(s): ES)

1. Procédé de préparation d'un fraqment d'ADN codant pour un polypeptide issu du cerveau humain, possédant une activité natriurétique et constitué par la séquence d'acides aminés suivante: 
Ser Pro Lys Met Val Gln Gly Ser Gly Cys Phe Gly
Arg Lys Met Asp Arg Ile Ser Ser Ser Ser Gly Leu Gly
Cys Lys Val Leu Arg Arg His
 lequel procédé est caractérisé en ce il comprend les étapes suivantes:
a) la construction d'une banque d'ADNc en menant les opérations suivantes: (1) isolement de l'ARNm à partir du cerveau ou de l'oreillette prélevés sur l'homme par homogénéisations de ces derniers, isolement par ultracentrifugation et purification; (2) synthèse de l'ADNc à partir de l'ARNm ainsi obtenu; (3) insertion d'un adaptateur de l'endonucléase EcoRI sur l'ADNc ainsi obtenu suivie de la phosphorylation de l'extrémité 5'; (4) ligature en utilisant un vecteur de manière à réaliser la construction de la banque;
b) le criblage du clone BNP humain à l'aide d'un fragment d'ADNc porcin marqué à titre de sonde ou hybridation de ceux-ci afin de sélectionner le clone positif;
c) le clivage du clone positif sélectionné avec une endonucléase convenable afin d'obtenir de fragment d'ADN recherché.

2. Procédé selon la revendication 1, caractérisé en ce que le fragment d'ADN présente la séquence de bases suivante:

AGC CCC AAG ATG GTG CAA GGG TCT GGC TGC TTT GGG AGG
AAG ATG GAC CGG ATC AGC TCC TCC AGT GGC CTG GGC TGC
AAA GTG CTG AGG CGG CAT.

3. Procédé de préparation d'un polypeptide physiologiquement actif représenté par la formule (I) 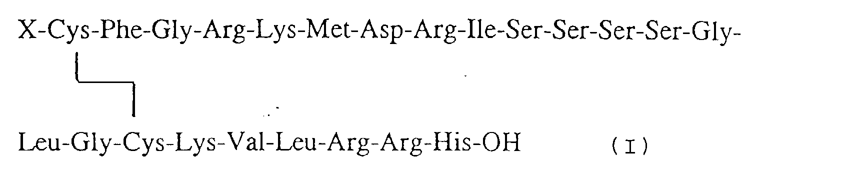
X-Cys-Phe-Gly-Arg-Lys-Met-Asp-Arg-Ile-Ser-Ser-Ser-Ser-Gly-
Leu-Gly-Cys-Lys-Val-Leu-Arg-Arg-His-OH (I)
 dans laquelle X est H, H-Gly-Ser-Gly-, ou H-Ser-Pro-Lys-Met-Val-Gln-Gly-Ser-Gly-,
lequel procédé est caractérisé en ce qu'il comprend:
a) la préparation d'un polypeptide par une technique de synthèse sur phase solide selon le mode suivant: (i) l'introduction d'abord du dérivé protégé Fmoc-His(Trt)-OH dans lequel His est l'acide aminé C-terminal du polypeptide dans la résine d'alcool p-alcoxybenzylique; (ii) la liaison successive des acides aminés protégés correspondants de manière à synthétiser un peptide protégé lié à la résine; (iii) le clivage ultérieur du polypeptide à partir de la résine et l'élimination des groupes protecteurs autres que Acm afin d'obtenir un peptide ayant un groupe Acm porté par le groupe thiol de la cystéine, un tel peptide étant désigné par Cys(Acm)-peptide; (iv) ensuite, l'oxydation du Cys(Acm)-peptide à l'iode afin d'éliminer le groupe protecteur du thiol et, la formation simultanée d'un pont disulfure entre les deux groupes thiol de cystéine au sein de la molécule peptidique, produisant ainsi un polypeptide brut de formule (i) indiquée ci-dessus, qui est purifié par la suite.
